# EUROPEAN PATENT APPLICATION

(11) **EP 3 821 900 A1**
(43) Date of publication of application: **19.05.2021**
(21) Application number: 20197039.9
(22) Date of filing: 18.09.2020
(51) Int. Cl.: A61K 36/185, A61K 36/35, A61K 36/45, A61K 36/58, A61K 36/605, A61K 36/886, A61K 36/9068, A61P 17/00, A61P 17/02

(54) **COMBINATION BROAD-SPECTRUM ANTIBIOTIC PREPARATIONS FOR DRUG-RESISTANT PATHOGENS**

(30) Priority: 15.11.2019 US 201916685529
(71) Applicant: Fenzl, Mark Edward, Sarasota, FL 34232 (US)
(72) Inventor: Fenzl, Mark Edward, Sarasota, FL 34232 (US)
(74) Representative: Sach, Greg Robert

(57) **Abstract**

Drug-resistant pathogens have led to disease and expense for health care, agriculture, and food industries. Synthetic chemicals have become less effective at killing antibiotic-resistant pathogens and there is not likely going to be the development of new antibiotics in the future. This disclosure provides for a combination broad-spectrum antibiotic using a combination of plant extracts that protect from drug-resistant pathogens.

## Description

### FIELD

The present disclosure relates to antibiotics and, more particularly, to antibiotic additives.

### BACKGROUND

There is a need to protect people and products from pathogens. Bacteria, yeast, and fungi cause disease, damage products, and damage food. Natural chemicals can inhibit and kill microbes that cause these problems. Historically salves were made by soaking herbs in oils or mixing them with beeswax. These were applied topically or to wounds. Food was preserved using spices. Prior to scientific method, there was no means to determine the effectiveness of salves or spices other than clinical experience. Without microscopes there was limited knowledge of microorganisms associated with disease. Cultivars of domesticated herbs, flowers, fruit, and berries were cultivated for their size and strength of their smell and taste. Chemical concentration was determined by taste and smell and often herbs, flowers, and fruit with a stronger scent were more likely to have stronger antibiotic properties.

Later, more effective synthetic products were developed to inhibit and kill pathogens. The problem is that synthetic antibiotics and preservatives can cause allergic reactions, have side effects, or promote cancer. Also, after the advent of synthetic antibiotics and preservatives, pathogens started developing resistance. According to Rosa Capita and Carlos Alonso-Calleja (2013) in Critical Reviews in Food Science and Nutrition, multidrug resistant bacteria cause half of healthcare-associated death from infections in Europe and cost between $4 billion and $5 billion in the US in 1995.

The extensive use of antibiotics by the meat industry will make the problem of antibiotic resistance worse in the future and there is a need to develop alternative means to protect animal feed and meat producing animals from antibiotic resistant microbes. Animals infected with antibiotic resistant pathogens have the potential to pass these diseases on to people. Not only are animals and meat susceptible to antibiotic resistant pathogens, but plant foods are also susceptible to pathogens that can cause food damage or infections in people.

Despite the need for new antibiotics to combat antibiotic resistant bacteria, the pharmaceutical industry is investing a very small percentage of their resources on the development of new antibiotics and the cost of developing new antibiotics is extremely expensive.

The pharmaceutical industry states it is unlikely that significant new antibiotics will be discovered in the future (Capita & Alonso-Calleja, 2013). One way to prevent these infections without the side effects of synthetic chemicals is to use natural nontoxic plant products that inhibit antibiotic resistant bacteria.

According to Mayaud, Carricajo, Zhiri, and Aubert (2008) in Applied Microbiology, antibiotic-resistant "methicillin-resistant *Staphylococcus aureus* (MRSA), vancomycin-resistant *Enterococcus* (VRE), *Pseudomonas aeruginosa, Acetobacter* and enterobacteria producing extended-spectrum β-lactamase (ESBL)" are a major worldwide health concern for nosocomial infections. Laura Pimentel (2005) in Relias Media states that common community-acquired antibiotic-resistant bacteria include β-Lactamase producing Escherichia coli, β-Lactamase producing Hemophilus Influenzae, β-Lactamase producing Moraxella Catarrhalis, methicillin-resistant Staphylococcus Aureus, and drug-resistant Streptococcus pneumoniae. Caroline DeWaal and Susan Grooters (2013) in the Center for Science in the Public Interest stated that antibiotic resistant *Salmonella,* enterotoxigenic *Escherichia coli* (ETEC), *Campylobacter,* and *Staphylococcus aureus* cause antibiotic-resistant foodborne outbreaks. Marta Maroszyriska, Anilna Kunicka-Styczynska, Katarzyna Rajkowska, and Iwona Maroszyriska (2013) in Acta Biochimica Polonica state that antibiotic-resistant strains of *candida albicans* are found in clinics, food, and beyond the hospital. I have invented combination preservatives and antibiotics that inhibit antibiotic-resistant organisms.

Some natural preparations containing capsaicin, salicylates, and hydrolysable tannins have antibiotic properties, but are allergenic, toxic, irritating to mucus membranes, or can burn the skin. Capsaicin, the chemical in hot peppers, can be extremely irritating to mucus membranes. Salicylates, including salicylic acid, found in wintergreen plants, white willow bark, and aspen bark, are potentially fatal. Tannins found in oak extract are used to tan hides and preserve wines. Tannins can cause dermatitis, allergic reactions, or intestinal dysfunction. These ingredients are used in some natural preparations, but because of these dangers are not used in my invention.

There are a variety of dietary supplements that contain concentrated natural products that have antibacterial or preservative properties but were not developed, manufactured, or sold for that purpose and do not cover a broad spectrum of pathogens. There are patents for natural preservatives and natural preservatives on the market, but they do not effectively inhibit or kill antibiotic-resistant microbes.

Another difficulty with using plant products for antibiotic preparations is that plant products that are resistant to some microorganisms are susceptible to other microorganisms and the nutrients and water found in plants feed those microorganisms. To avoid these problems, I have combined antibiotic plant products that inhibit or kill a variety of microorganisms that are resistant to antibiotics to provide broad spectrum coverage.

Another way to inhibit microbial growth is limiting the amount of water in the product by using concentrated plant products and / or admixing the plant products with plant materials that lower water activity level. Water activity level is the amount of free water in a product. Examples of materials that lower water activity level include glycerol or sorbitol. These materials bind to water to prevent the availability of free water that bacteria need to grow and multiply.

Healthcare and veterinary costs are rising. The cost of food will rise with the spread of antibiotic-resistant food pathogens. The invention can provide the ability to effectively combat antibiotic-resistant bacteria without the exorbitant cost of creating new chemicals or using toxic synthetic preservatives. Society wants chemical-free products and yet the risk of antibiotic-resistant bacteria in the hospital and the community is on the rise and will continue to rise in the future. Farmers have been providing live-stock with antibiotics in feed to increase weight and protect from infection, but this has led to an increase in antibiotic-resistant bacteria on the farm, in the grocery store, in the hospital, and in the community. Not only can the invention protect farmers and animals from infections found on the farm, but also protect feed and food products from damage. Using a combination of natural antibiotics and preservatives is an economical and practical solution for people, animals, and food without exposing microorganisms to a new chemical that will induce resistance.

There is a continuing need for a broad-spectrum antibiotic preparations that inhibit antibiotic-resistant microbes. Desirably, the antibiotic preparation is manufactured by combining concentrated, non-synthetic plants products.

### SUMMARY

In concordance with the instant disclosure, a broad-spectrum antibiotic preparations that inhibit antibiotic-resistant microbes that is manufactured by combining concentrated, non-synthetic plants products, has surprisingly been discovered.

Certain natural ingredients have been found useful for militating against bacterial growth when provided in appropriate concentrations. Additives made from combinations of these natural ingredients are useful for supplying these ingredients in effective amounts, especially when prevention of bacterial and fungal growth is desirable.

Synthetic antibiotics and preservatives have been effective at inhibiting and killing bacteria at lower concentrations than plant products. One problem with synthetic antibiotics and preservatives is that they can cause allergic reactions, toxicity, or promote cancer. Also, some bacteria have developed resistance to synthetic antibiotics and preservatives, rendering them ineffective. This has created a huge expense in health care, agriculture, and food industries. This new, progressive, systemic problem requires a unique, scientific approach for a solution.

In one embodiment, an antibiotic additive may be configured to be admixed in a product. The antibiotic additive may include at least four ingredients selected from at least two of a plurality of groups. The groups include: a first group, the first group containing carvacrol, cineole, cinnamaldehyde, citral, citronellal, eugenol, geraniol, linalool, limonene, perillaldehyde, pinene, sabinene, terpineols, thujone, and thymol, wherein each of the ingredients of the first group are present at a concentration of 250 and 25,000 parts per million in the product; a second group, the second group containing anethole, berberine, catechins, chebulic acid, ferulic acid, flavonols, glycyrrhetinic acid, hesperidin, isothiocyanates, mangostin, piperine, pterostilbene, theaflavins, and withanolides wherein each of the ingredients of the second group are present at a concentration of between 500 parts per million and 50,000 parts per million in the product; a third group, the third group containing bearberry extract, bilberry extract, elderberry extract, and mulberry extract. Each of the ingredients of the third group are present at a concentration of between 1,000 parts per million and 100,000 parts per million in the product; and a fourth group, the fourth group containing aloe vera juice, cocoa, ginger juice, molasses, neem oil, and honey at a concentration of 10,000 parts per million in the product.

In another embodiment, the antibiotic additive may be configured to be admixed in a product. The antibiotic additive includes at least three ingredients selected from each of a plurality of groups. The groups include: a first group, the first group containing carvacrol, cineole, cinnamaldehyde, citral, citronellal, eugenol, geraniol, linalool, limonene, perillaldehyde, pinene, sabinene, terpineols, thujone, and thymol, wherein each of the ingredients of the first group are present at a concentration of 250 and 25,000 parts per million in the product; a second group, the second group containing bearberry extract, bilberry extract, elderberry extract, and mulberry extract, wherein each of the ingredients of the second group are present at a concentration of between 1,000 parts per million and 100,000 parts per million in the product; and a third group, the third group containing aloe vera juice, cocoa, ginger juice, molasses, neem oil, and honey at a concentration of 10,000 parts per million in the product.

In a third embodiment, the antibiotic additive may be configured to be admixed in a product. The antibiotic additive includes at least three ingredients selected from each of a plurality of groups, the groups including the first group, the first group containing anethole, berberine, catechins, chebulic acid, ferulic acid, flavonols, glycyrrhetinic acid, hesperidin, isothiocyanates, mangostin, piperine, pterostilbene, theaflavins, and withanolides wherein each of the ingredients of the second group are present at a concentration of between 500 parts per million and 50,000 parts per million in the product; a second group, the second group containing bearberry extract, bilberry extract, elderberry extract, and mulberry extract, wherein each of the ingredients of the second group are present at a concentration of between 1,000 parts per million and 100,000 parts per million in the product; and a third group, the third group containing aloe vera juice, cocoa, ginger juice, molasses, neem oil, and honey at a concentration of 10,000 parts per million in the product.

### DETAILED DESCRIPTION

The following description is merely exemplary in nature and is not intended to limit the present disclosure, application, or uses. In respect of the methods disclosed, the order of the steps presented is exemplary in nature, and thus, is not necessary or critical unless otherwise disclosed.

The following plant species have been found to inhibit or kill drug resistant bacteria: *Acinetobacter baumannii, Bacillus subtilis, Burkholderia cepacia* complex, *Candida albicans, Enterococcus, Escherichia coli, Klebsiella pneumonia, Mycobacterium tuberculosis, Proteus vulgaris, Pseudomonas aeruginosa, Salmonella typhi, Shigella sonnei, Staphylococcus aureus, Staphylococcus epidermis,* or *Vibrio cholera.*

It has been found that rosemary essential oil inhibits four strains of multidrug resistant bacteria at 5% concentration and clove essential oil inhibited four strains of multidrug resistant bacteria at 1.25% concentration. A chemical constituents of rosemary essential oil is cineole. The main chemical constituent of clove essential oil is eugenol.

It has been found that *A. lebbeck, T. chebula, S. cumini, B. monosperma, S. indica, A. marmelos,* and *W. somnifera* kill four strains of multi-drug-resistant *V. cholera* at 24 milligrams per milliliters. *A. lebbek* contains the antibiotic catechin. *T. Cheluba* contains the antibiotic product chebulic acid. *W. somnifera* contains a class of antibiotic products called withanolides.

It has been found that lemongrass essential oil and citral kills methicillin-resistant *Staphylococus aureus* at a concentrations of 0.13% and 0.06%. Citral is the most abundant chemical in lemongrass essential oil.

It has been found that extracts from *Acorus calamus, Camellia sinensis, Cichorium intybus, Delonix regia, Hemidesmus indicus, Holarrhena antidysenterica, Lawsania intermis, Mangifera indica, Plumbago zeylanica, Punica granatum, Terminalia bellerica, and Terminalia cheluba,* inhibited multidrug resistant *Escheria coli* in concentrations of 10.24 milligrams per milliliter.

The essential oil from *Acorus calamus* contains gurjunene. *Camellia sinensis* contains the antibiotic products chatechins and theaflavin. *Cichorium intybus* essential oil contains thymol, cymene, and carvacrol. *Delonix regia* contains the antibacterial products benzoic acid, quercetin, rutin, and kaempferol. *Terminalia cheluba* contains the antibiotic product chebulic acid.

It has been found that black seed oil inhibits thirty isolates of multidrug resistant *Pseudomonas aerginosa* at a concentration of 1:25.

It has been found that chasteberry extract inhibited methicillin-resistant *Staphylococcus aureus* at a concentration of 0.312 milligrams per milliliter. Chasteberry essential oil contains the antibiotic products sabinene.

It has been found that the following compounds have demonstrated antibiotic properties and inhibit MRSA: isothiocyanates.

It has been found that pomegranate molasses inhibits multidrug resistant *Serratia marcescen.*

It has been found that ethanolic extracts of anise, coriander, dill, rosmarinus, marjoram, lavender, thyme, chamomile, lantana, guava, carob gum, licorice, lemon grass, basil, and oregano inhibit methicillin-resistant *Staphylococcus aureus.* The main chemical constituent of anise is anethole.

It has been found that theaflavin inhibits six strains of multidrug resistant *Acinetobacter baumannii* at a concentration of 512 milligrams per liter and epicatechin inhibits six strains of multidrug resistant *Acinetobacter baumannii* at a concentration of 2048 milligrams per liter.

It has been found that linalool, geraniol, and basil essential oil inhibit antibiotic resistant *C*. *albicans* at a concentration of 1580 micrograms per milliliter.

It has been found that pinene inhibits antibiotic resistant *Staphylococcus aureus* at a concentration of 20 microliters per milliliter.

It has been found that ethanolic extracts of aloe vera, neem, bryophyllum, lemongrass, tulsi, oregano, rosemary, and thyme inhibit multi-drug resistant *Klebsiella pneumoniae* at a concentration of 12.5 milligrams per milliliter.

It has been found that methanolic extract of *Terminalia arjuna* inhibit 13 strains of multidrug resistant bacteria at a concentration of 1.28 milligrams per milliliter.

It has been found that methanol extract of pomegranate kills 10 stains of *Mycobacterim tuberculosis,* including six strains that are resistant to antibiotics, in concentrations of 1024 micrograms per milliliter. It has also been shown that the polyphenolic constituents of pomegranate, caffeic acid, EGCG, and quercetin kill ten strains of *Mycobacterim tuberculosis* and twenty strains of multidrug resistant *Klebsiella pneumonia* at a concentration of 1024 microgram per milliliter.

It has been found that ginger extract, and thyme essential oil inhibit five strains of multi-drug resistant *Pseudomonas aeruginosa* in concentrations of 80 milligrams per milliliter, 0.96 milligrams per milliliter, and 0.48 milligrams per milliliter respectively.

It has been found that *Juglans regia* extract inhibits methicillin resistant *Staphylococcus aureus* at a concentration of 0.31 milligrams per milliliter.

It has been found that guava extract kills three strains of multidrug resistant bacteria at 312.5 milligrams per milliliter and that catechin, epicatechin, chlorogenic acid, ferulic acid, quercetin, luteolin, and kaempferol kill four antibiotic resistant bacteria at concentrations of 5 milligrams per milliliter.

It has been found that *Aloe vera* gel inhibits multidrug-resistant *Pseudomonas aerginosa* at a concentration of 200 micrograms per milliliter.

It has been found that using garlic and ginger extracts in concentration of 1 milligram per milliliter results in inhibition of multiple drug resistant *Escherichia coli, Pseudomonas aeruginosa, Bacillus subtilis, Staphylococcus aureus, Klebsiella pneumonia, Shigella sonnei, Staphylococcus epidermis,* and *Salmonella typhi.*

It has been found that thyme tea, wormwood tea, mint tea, rosehip tea, pomegranate tea, black tea, green tea, orange tea, and sage teas inhibit Methicillin Resistant *Staphylococcus aureus* in a concentration of 1.25% and cinnamon tea inhibits multidrug resistant *Candida albicans* at a concentration of 2.5%. Sage contains the antibiotic product thujone.

It has been found that 20 strains of multidrug resistant *Pseudomonas aeruginosa* were killed at a concentration of 30 milligrams per liter of thyme essential oil. All strains were inhibited by *Aloe vera* and *Zararia multiflora* extracts at a concentration of 20 milligrams per milliliter.

It has been found elder flower and elderberry have antimicrobial properties against MRSA.

It has been found that aniseed extract and star anise extract inhibit 100 strains of drug resistant bacteria at a concentration of 128 micrograms per milliliter.

It has been found that cinnamon, clove, tree basil, sweet basil, lemongrass, plai, lime, ginger, kaffir lime, and tea tree essential oils inhibit multi-drug resistant *Acinetobacter baumannii* at a concentration of 4 milligrams per milliliter.

It has been found that aqueous extracts of *Berberis vulgaris* inhibit 140 strains of multidrug-resistant gram-negative bacteria at a concentration of 50 milligrams per milliliter. *Berberis vulgaris* contains the antibiotic products berberine.

It has been found that methanolic extracts of *Artocarpus heterophyllus, Elettaria cardamomum, Moringa oleifera,* and *Tamarindus indica* inhibit multidrug resistant methicillin resistant *Staphylococcus aureus* at 30 milligrams per milliliter.

It has been found that garlic and ginger extracts inhibited multiple strains of multidrug resistant bacteria at concentrations of 160.2 micrograms per milliliter and 185.58 micrograms per milliliter respectively.

It has been found that ethanolic extracts of *Acacia nilotica, Syzygium aromaticum,* and *Cinnamum zeylanicum* inhibit multiple strains of multidrug resistant bacteria at 1560 micrograms per milliliter.

It has been found that isothiocyanates, including phenylethyl isothiocyanate, extracted from horseradish kill four strains of antibiotic resistant bacteria at a concentration of 2000 microgram per milliliter.

It has been found that glycerrhetinic acid, found in licorice root, kills methicillin-resistant *Staphylococcus aureus* at a 0.223 micromolar concentration.

It has been found that aqueous ginger extracts inhibited multidrug resistant *Bacillus* subtilis and *Pseudomonas aeruginosa* at concentrations of 50 milligrams per milliliter.

It has been found that lemon eucalyptus essential oil kills ten strains of multidrug resistant *Candida albicans* at concentrations of 10 milligrams per milliliter. The main constituent of lemon eucalyptus essential oil is citronellal.

It has been found that carvacrol inhibits 32 strains of antibiotic-resistant streptococcus at a concentration of 256 micrograms per milliliter, thyme and oregano inhibited 32 strains of antibiotic-resistant streptococcus at a concentration of 512 micrograms per milliliter and peppermint essential oil inhibited 32 strains of antibiotic-resistant streptococcus at a concentration of 2048 micrograms per milliliter.

It has been found that rosemary, fennel, and galbanum essential oils inhibit eight strains of methicillin resistant *Staphylococcus aureus* in concentration of 16 microliters per milliliter.

It has been found that cinnamon bark, oregano, ajowan, thyme, clove, cinnamon leaf, lemon grass, tea tree, palmarosa, eucalyptus, cajeput, and lavender essential oils inhibit antibiotic-resistant *Acinetobacter baumanii* at a concentration of 5.27% or less. The main chemical constituent of palmarosa essential oil is geraniol.

It has been found that norwogonin, trechebulin, chebulagic acid, and corilagin inhibit multidrug-resistant *Acinetobacter baumannii* at a concentration of 1,000 micrograms per milliliter.

It has been found that mulberry extract is bactericidal of *Staphylococcus aureus, Pseudomonas aeruginosa, Escherichia coli,* and *Salmonella typhi* at a concentration of 1.28 milligrams per milliliter and inhibits antibiotic resistant *Staphylococcus aureus,* antibiotic resistant *Escherichia coli,* and antibiotic resistant *Salmonella typhi.*

It has been found that piliostigmol inhibits *Staphylococcus aureus* (NCTC 6571), *Escherichia coli* (NCTC 10418), *Bacillus subtilis* (NCTC 8236), *Proteus vulgaris* (NCTC 4175), *Aspergillus niger* (ATCC 10578) and *Candida albicans* (ATCC 10231).

It has been found that pterostilbene kills methicillin resistant *Staphylococcus aureus at* a *concentration of 0.156 millimoles.*

It has been found that basil essential oil inhibits *Escherichia coli* that is resistant to tetracycline in the concentration of 18 micrograms per milliliter.

It has been found that clove oil, thyme oil, and origanum oil inhibited multidrug resistant *Pseudomonas aeruginosa* and *Burkholderia cepacia* complex isolates at a concentration of 10⁻² microliters per milliliter.

It has been found that thyme essential oil, clove essential oil, and jambolan extract inhibited antibiotic resistant *Pseudomonas aerginosa;* lemonbalm essential oil inhibit antibiotic-resistant *Klebsiella pneumonia.*

It has been found that glycyrrhetic acid, kills 18 strains of methicillin resistant *Staphylococcus aureus* at concentrations of 512 milligrams per milliliter.

It has been found that *Holarrhea antidysenterica, Punica granatum, Quisqualis indica, terminalia bellrica, Terminalia chebula,* and *Terminalia sp.* inhibited multidrug resistant *Acinetobacter baumannii* at a concentration of 125 micrograms per milliliter.

It has been found that green tea inhibits methicillin-resistant *Staphylococcus aureus* and multi-drug resistant *Pseudomonas aeruginosa* at a concentration of 400 micrograms per milliliter and 800 micrograms per milliliter.

It has been found that honey is fungicidal of fluconazole-resistant *Candida albicans* at a concentration of 25% to 56.25%.

It has been found that *Theobroma* cacao extracts kill antibiotic resistant *Escherichia coli* at a concentration of 64 micrograms per milliliter.

It has been found that 19 spices, *C. zeylanicum, C. longa, M. koenigii, A. cepa, B. juncea, C. cyminum, C. tamala, C. melo,* C. *amada, F. vulgare, S. aromaticum, T. ammi, Z. officinale, A. sativum, C. annuum, I. verum, M. spicata, M. fragrans, and P. nigru* each inhibit at least one strain of multidrug resistant bacteria. The main chemical constituent of *M*. *spicata* is carvone and an antibiotic product of *M*. *spicata* is perillaldehyde. Piperine is in tumeric.

It has been found that basil, origanum, tea tree, and thyme oils kill 20 strains of multidrug resistant bacteria at a concentration of 4%.

It has been found that aqueous extracts of aster inhibit six strains of multidrug resistant bacteria at a concentration of 10 milligrams per milliliter and ethanolic extract of calendula inhibited six strains of multidrug resistant bacteria at a concentration of 10 milligrams per milliliter.

It has been found that thyme essential oil inhibits 120 strains of multidrug resistant bacteria at a concentration of 2.5 microliters per milliliter.

It has been found that geranium essential oil inhibits 75 strains of antibiotic resistant bacteria at a concentration of 10.5 microliters per milliliter.

It has been found that *Holarrhena antidysenterica* extract inhibits extensively multidrug resistant *Acinetobacter baumannii* at a concentration of 1,250 milligrams per liter and conessine inhibited extensively multidrug resistant *Acinetobacter baumannii* at a concentration of 10 milligrams per liter.

It has been found that F. *indica, L. inermis, P*. *oleracea* and *Z*. spina-Christi extracts inhibit multidrug resistant *A. baumanni* at a concentration of 25 micrograms per milliliter. *P*. *oleracea* contains hesperidin.

It has been found that 10% ethanolic extract of *Garcinia mangostana* inhibited methicillin-resistant *Staphylococcus aureus. Garcinia mangostana* contains mangostin.

It has been found that thyme essential oil inhibits 59 strains of multidrug resistant *Acinetobacter baumannii* at a concentration of 2 micrograms per milliliter.

It has been found that *Psidium guava, Phyllanthus niruri, Ehretia microphylla,* and *Piper betle* inhibit methicillin-resistant *Staphylococcus aureus* and vancomycin-resistant *Enterococcus.*

It has been found that aqueous extracts of bearberry leaves kill *Escherichia coli* and *Enterococcus faecalis* strains that are resistant to amoxicillin in the concentration of 5 mg /ml.

It has been found that aqueous bilberry extract kills 10 strains of *E*. *coli,* seven which are resistant to amoxicillin at 40 milligrams per milliliter. Aqueous bilberry extract also kills 10 strains of *P*. *vulgaris,* eight of which are resistant to amoxicillin, at concentrations of 40 milligrams per milliliter.

It has been found that epicatechin, catechin, caffeine, chlorogenic acid, theobromine, theophylline, quercitin, kampferol, gallocatechin, epigallocatechin, epigallocatechin gallate, catechin gallate, epicatechin gallate, epiafzelechin, epiafzelechin gallate, theasinesin A, theaflavin, theaflavin gallate, and theaflavin digallate have antibiotic properties against antibiotic-resistant bacteria.

It has been found that ethanol extract of *Dracontomelon* Dao kills methicillin-resistant *Staphylococcus aureus* and multiple drug resistant *Escherichia coli.* The Pacific Walnut contains catechin.

The natural ingredients described hereinabove may be combined to create an additive. The additive may be placed in a product, on a product, or admixed with a product. The additive may inhibit *Candida,* inhibit antibiotic-resistant *Staphylococcus,* inhibit antibiotic-resistant *Escherichia coli,* and inhibit antibiotic-resistant *Pseudomonas.* The product may be one of a cosmetic preservative, food preservative, or pharmaceutical preservative. It should be understood that a skilled artisan may utilize this additive in any suitable product, in which, antibiotic properties would be desired.

It should be appreciated that the additive of the present disclosure may be admixed with a variety of products. As non-limiting examples, the additive may be formulated for wound application, epidermal application, nasal application, otic application, mucus membrane application, *H. pylori* application, pharmaceutical application, veterinary application, food preservation, and animal feed preservation. A skilled artisan may admix the additive into suitable formulations where a natural antibiotic is desired.

The additive may include natural antimicrobial products selected from a plurality of groups. The first group includes terpenoids found in essential oils. The second group includes polyphenols found in plants. The third group includes anthocyanins found in dark fruit. The fourth group includes plant extracts that contain a broad combination of natural antimicrobials. Advantageously, using antimicrobials from different groups ensures that the antimicrobials inhibit microbes in a variety of ways.

In one embodiment, the antibiotic additive may be configured to be admixed in the product. The antibiotic additive may include at least four ingredients. The ingredients may be selected from at least two of a first plurality of groups, described hereinbelow.

A first group may contain carvacrol, cineole, cinnamaldehyde, citral, citronellal, eugenol, geraniol, linalool, limonene, perillaldehyde, pinene, sabinene, terpineols, thujone, and thymol. Each of the ingredients of the first group may be present at a concentration between 250 and 25,000 parts per million in the product.

A second group may contain anethole, berberine, catechins, chebulic acid, ferulic acid, flavonols, glycyrrhetinic acid, hesperidin, isothiocyanates, mangostin, piperine, pterostilbene, theaflavins, and withanolides. Each of the ingredients of the second group may be present at a concentration between 500 parts per million and 50,000 parts per million in the product.

A third group may contain bearberry extract, bilberry extract, elderberry extract, and mulberry extract. Each of the ingredients of the third group may be present at a concentration between 1,000 parts per million and 100,000 parts per million in the product.

A fourth group may contain aloe vera juice, cocoa, ginger juice, molasses, neem oil, and honey. Each of the ingredients of the fourth group may be present at a concentration of more than 10,000 parts per million in the product.

It should be appreciated that a skilled artisan may select a suitable combination of ingredients at suitable concentrations, as desired.

In another embodiment, the antibiotic additive may be configured to be admixed in the product. The antibiotic additive may include at least three ingredients selected from each of a second plurality of groups, as described hereinbelow.

A first group may contain carvacrol, cineole, cinnamaldehyde, citral, citronellal, eugenol, geraniol, linalool, limonene, perillaldehyde, pinene, sabinene, terpineols, thujone, and thymol. Each of the ingredients of the first group may be present at a concentration between 250 and 25,000 parts per million in the product.

A second group may contain bearberry extract, bilberry extract, elderberry extract, and mulberry extract. Each of the ingredients of the second group may be present at a concentration of between 1,000 parts per million and 100,000 parts per million in the product.

A third group may contain aloe vera juice, cocoa, ginger juice, molasses, neem oil, and honey. Each of the ingredients of the third group may be present at a concentration of more than 10,000 parts per million in the product.

In a third embodiment, the antibiotic additive may be configured to be admixed in the product. The antibiotic additive may include at least three ingredients selected from each of a third plurality of groups, as described hereinbelow.

A first group may contain anethole, berberine, catechins, chebulic acid, ferulic acid, flavonols, glycyrrhetinic acid, hesperidin, isothiocyanates, mangostin, piperine, pterostilbene, theaflavins, and withanolides. Each of the ingredients of the second group may be present at a concentration of between 500 parts per million and 50,000 parts per million in the product.

A second group may contain bearberry extract, bilberry extract, elderberry extract, and mulberry extract. Each of the ingredients of the second group may be present at a concentration of between 1,000 parts per million and 100,000 parts per million in the product.

A third group may contain aloe vera juice, cocoa, ginger juice, molasses, neem oil, and honey. Each of the ingredients of the third group may be present at a concentration of more than 10,000 parts per million in the product.

A skilled artisan may select a suitable combination of ingredients at suitable concentrations, as desired.

In a most particular embodiment, the product may be a topical wound preparation such as a wound honey or wound gel, as non-limiting examples. The topical wound preparation contains honey. It should be appreciated that honey lowers the water activity level of the product. The topical wound preparation may further include aloe vera juice with aloe vera extract, elderberry extract, licorice root extract with glycyrrhizic acid, clove essential oil with eugenol, and oregano essential oil with carvacrol. It should be appreciated that these ingredients are added to make the topical wound preparation fungicidal and bactericidal.

This combination of natural antibiotics provides multiple coverage against antibiotic-resistant pathogens. For example, clove essential oil and oregano essential oil may each be provided at concentrations that inhibit antibiotic-resistant candida albicans. Glycyrrhizic acid and eugenol may each be in a concentration sufficient to be bactericidal of Methicillin-resistant *Staphylococcus aureus.* Eugenol and carvacrol have a synergistic effect on methicillin-resistant *Staphylococcus* aureus, as shown in Table 1 below. Elderberry extract, glycyrrhizic acid, and aloe vera gel are in a concentration sufficient to be bactericidal of antibiotic-resistant *Pseudomonas aeruginosa.*

**Table 1**

| **MIC / MBC / MBEC of Carvacrol and Eugenol for MRSA** | | | |
|---|---|---|---|
| | MIC | MBC | MBEC |
| Carvacrol | 0.02 | | 0.04 |
| Eugenol | 0.04 | 0.08 | 0.08 |
| Carvacrol / Eugenol | | | 0.01/0.02 |

| | | | |
|---|---|---|---|
| MIC - Minimal Inhibitory Concentration, MBC- Minimal Bactericidal Concentration, MBEC - Minimal Biofilm Eradication Concentration. Concentrations in percentage of volume. | | | |

Oregano essential oil and clove essential oil inhibit Escheria coli, Pseudomonas aeruginosa, and Staphylococcus aureus, as shown in Table 2 below.

**Table 2**

| **MBC of Oregano Oil and Clove Oil** | | | |
|---|---|---|---|
| Oil | ***E. coli*** | ***P. aeruginosa*** | ***S*. *Aureus*** |
| Oregano | 0.097 | 0.781 | 0.097 |
| Clove | 0.390 | 3.125 | 0.781 |

Eugenol and carvacrol inhibit Candida Albicans, as shown in Table 3 below.

**Table 3**

| **MBC of Carvacrol and Eugenol against *Candida Albicans*** | | | | |
|---|---|---|---|---|
| Strain of Candida *albicans* | *ATC 00928NCPF 3153* | | | Ca2 |
| Carvacrol | 0.5 | 0.5 | 0.5 | |
| Eugenol | 0.12 | 0.12 | 0.12 | |

Different ingredients have different mechanisms for killing microbes, whether it is damage to the cell membrane, damage to cell organelles, inhibition of replication, or damage to cell DNA. Thus, the topical wound preparation provides antibacterial protection by limiting different materials that are needed for microbial growth and using antimicrobial agents that are microbiocidal using a variety of mechanisms.

Another way to inhibit microbial growth is limiting the amount of water in the product by using concentrated plant products or admixing the plant products with plant materials that lower water activity level. Water activity level is the amount of free water in a product. Examples of materials that lower water activity level include glycerol or citric acid. These materials have been found to bind to water to prevent the availability of free water that bacteria need to grow and multiply.

Water activity may be defined as the ratio of the vapor pressure of water in a substance to the vapor pressure of pure water at the same temperature. Microorganisms require a minimum level of water activity in order to grow and, thus, water activity reduction can be used to control microbial growth.

Accordingly, it is desirable for the additive to have a low water activity level. A humectant may be used to reduce the water activity level of the additive. The humectant may bind to the free water molecules in the additive and thereby reduce the water activity level. As non-limiting examples, the humectant may be nitric acid, dextrose, fructose, glycerol, glycine, glucose, honey, malic acid, salt, sorbitol, sucrose, and tartaric acid. A skilled artisan may select any suitable humectant, as desired.

As may be presented herein, the language "consisting essentially of" is meant to limit the scope of the claim to the specified materials that do not materially affect the basic and novel characteristics of the additive. It should be appreciated that ingredients that materially affect the additive may improve or adversely affect the antimicrobial properties of the additive. Thus, the additive consisting essentially of certain ingredients described hereinabove, excludes ingredients that may improve or adversely affect the antimicrobial properties of the additive, which would materially affect the basic and novel characteristics of the additive.

Additionally, as may be presented in the claims below, the language "consisting of" is intended to exclude any ingredient not specified in the claim. Accordingly, the additive consisting of certain ingredients described hereinabove includes only those ingredients.

While certain representative embodiments and details have been shown for purposes of illustrating the invention, it will be apparent to those skilled in the art that various changes may be made without departing from the scope of the disclosure, which is further described in the following appended claims.

## Claims

1. An antibiotic additive configured to be admixed in a product, the antibiotic additive comprising:
at least four ingredients selected from at least two of a plurality of groups, the groups including:
a first group, the first group containing carvacrol, cineole, cinnamaldehyde, citral, citronellal, eugenol, geraniol, linalool, limonene, perillaldehyde, pinene, sabinene, terpineols, thujone, and thymol wherein each of the ingredients of the first group are present at a concentration of between 250 and 25,000 parts per million in the product;
a second group, the second group containing anethole, berberine, catechins, chebulic acid, ferulic acid, flavonols, glycyrrhetinic acid, hesperidin, isothiocyanates, mangostin, piperine, pterostilbene, theaflavins, and withanolides wherein each of the ingredients of the second group are present at a concentration of between 500 parts per million and 50,000 parts per million in the product;
a third group, the third group containing bearberry extract, bilberry extract, elderberry extract, and mulberry extract wherein each of the ingredients of the third group are present at a concentration of between 1,000 parts per million and 100,000 parts per million in the product; and
a fourth group, the fourth group containing aloe vera juice, cocoa, ginger juice, molasses, neem oil, and honey wherein each of the ingredients of the fourth group are present at a concentration of more than 10,000 parts per million in the product.

2. The additive of Claim 1, further comprising a combination of 50% water activity reducing agents selected from the list of citric acid, dextrose, fructose, glycerol, glycine, glucose, malic acid, salt, sorbitol, sucrose, and tartaric acid.

3. The additive of Claim 1, wherein the additive has a water activity level is less than 0.85.

4. The additive in Claim 1, wherein the additive is admixed in the product.

5. The additive of Claim 1, wherein the product is one of a cosmetic, a food, and a pharmaceutical.

6. An antibiotic additive configured to be admixed in a product, the antibiotic additive comprising:
at least three ingredients each selected from one of three plurality groups, the groups including:
a first group, the first group containing carvacrol, cineole, cinnamaldehyde, citral, citronellal, eugenol, geraniol, linalool, limonene, perillaldehyde, pinene, sabinene, terpineols, thujone, and thymol wherein each of the ingredients of the first group are present at a concentration of between 250 and 25,000 parts per million in the product;
a second group, the second group containing bearberry extract, bilberry extract, elderberry extract, and mulberry extract wherein each of the ingredients of the second group are present at a concentration of between 1,000 parts per million and 100,000 parts per million in the product; and
a third group, the third group containing aloe vera juice, cocoa, ginger juice, molasses, neem oil, and honey wherein each of the ingredients of the third group are present at a concentration of more than 10,000 parts per million in the product.

7. The additive of Claim 6, wherein the additive has a water activity level is less than 0.85.

8. The additive in Claim 6, wherein the additive is admixed in the product.

9. The additive of Claim 6, wherein the product is one of a cosmetic, a food, and a pharmaceutical.

10. An antibiotic additive configured to be admixed in a product, the antibiotic additive comprising:
at least three ingredients each selected from one of three plurality groups, the groups including:
a first group, the first group containing anethole, berberine, catechins, chebulic acid, ferulic acid, flavonols, glycyrrhetinic acid, hesperidin, isothiocyanates, mangostin, piperine, pterostilbene, theaflavins, and withanolides wherein each of the ingredients of the first group are present at a concentration of between 500 parts per million and 50,000 parts per million in the product;
a second group, the second group containing bearberry extract, bilberry extract, elderberry extract, and mulberry extract wherein each of the ingredients of the second group are present at a concentration of between 1,000 parts per million and 100,000 parts per million in the product; and
a third group, the third group containing aloe vera juice, cocoa, ginger juice, molasses, neem oil, and honey wherein each of the ingredients of the third group are present at a concentration of more than 10,000 parts per million in the product.

11. The additive of Claim 10, further comprising a combination of 50% water activity reducing agents selected from the list of citric acid, dextrose, fructose, glycerol, glycine, glucose, malic acid, salt, sorbitol, sucrose, and tartaric acid.

12. The additive in Claim 10, wherein the additive is admixed in the product.

13. The additive of Claim 10, wherein the product is one of a cosmetic, a food, and a pharmaceutical.
